# FASCICULE DE BREVET EUROPEEN

(11) **EP 2 273 955 B1**
(45) Date de publication et mention de la délivrance du brevet: **20.08.2014**
(21) Numéro de dépôt: 09742318.0
(22) Date de dépôt: 10.04.2009
(51) Int. Cl.: A61F 5/00

(54) **ANNEAU GASTRIQUE COMPRENANT UNE CEINTURE MONOBLOC**
MAGENBAND MIT EINEM EINTEILIGEN GÜRTEL
GASTRIC RING INCLUDING A ONE-PIECE BELT

(30) Priorité: 14.04.2008 FR 0802033
(43) Date de publication de la demande: 19.01.2011
(73) Titulaire: Compagnie Europeenne d'Etude et de Recherche de Dispositifs pour l'Implantation par Laparoscopie, 38200 Vienne (FR)
(72) Inventeur: PAGANON, Pascal, F-69360 Serezin du Rhône (FR)
(74) Mandataire: Guérin, Jean-Philippe
(86) Numéro de dépôt international: PCT/FR2009/050670
(87) Numéro de publication internationale: WO 2009/136120

(56) Documents cités:
- EP-A- 0 028 962
- EP-A- 0 611 561
- EP-A- 1 829 504
- FR-A- 2 799 118
- FR-A- 2 834 631

## Description

### DOMAINE TECHNIQUE

La présente invention se rapporte au domaine technique général des implantes chirurgicaux destinés à être implantés dans le corps d'un patient autour d'un organe biologique constituant une poche ou un conduit.

La présente invention se rapporte notamment aux implants de types anneaux destinés à former une boucle fermée autour d'un organe biologique constituant une poche ou un conduit pour réduire le diamètre dudit organe biologique.

Le document EP0028962 décrit un sphincter artificiel formé d'une bande flexible présentant une série de chambre. Les chambres ont des parois hermétiques flexibles et st interconnectées. Une des chambres est connectée à un conduit à travers lequel on introduit ou retire du fluide au moyen d'une pompe. On réalise ainsi le remplissage ou le vidage des différentes chambres et ainsi leur gonflage et dégonflabe. La bande est disposée autour de l'intestin à proximité du stoma, sur la face extérieure de celui-ci. La pompe est actionnée manuellement. Lors du gonflement, les chambres se déforment pour occuper un espace intermédiaire entre elles jusqu'à venir en contact.

La présente invention concerne préférentiellement un anneau de gastroplastie, mais elle vise également un anneau conçu pour être utilisé dans le traitement de l'incontinence urinaire ou fécale (sphincter artificiel) ou encore un anneau conçu pour régler le débit sanguin dans des vaisseaux sanguins, cette liste n'étant nullement limitative.

La présente invention concerne plus particulièrement un anneau chirurgical implantable dans un corps humain ou animal, ledit anneau étant destiné à venir entourer un organe biologique constituant une poche ou un conduit en vue de modifier la section de passage dudit organe, ledit anneau comprenant d'une part une ceinture comportant au moins un moyen de verrouillage dudit anneau en position fonctionnelle fermée autour dudit organe biologique, et d'autre part une bande gonflable souple comprenant au moins une chambre de serrage annulaire destinée à recevoir un fluide de gonflage et comportant au moins une ouverture permettant l'apport en son sein du fluide de gonflage, ladite ceinture étant rapportée contre la bande gonflable souple et fixée à cette dernière.

La présente invention concerne également un procédé de fabrication d'un anneau chirurgical implantable dans un corps humain ou animal, ledit anneau étant destiné à venir entourer un organe biologique constituant une poche ou un conduit en vue de modifier la section de passage dudit organe, ledit procédé comprenant une étape de fabrication d'une ceinture comportant au moins un moyen de verrouillage dudit anneau en position fonctionnelle fermée autour dudit organe biologique, une étape de fabrication d'une bande gonflable souple comprenant au moins une chambre de serrage annulaire destinée à recevoir un fluide de gonflage et comportant au moins une ouverture permettant l'apport en son sein du fluide de gonflage, ainsi qu'une étape d'assemblage de la ceinture avec la bande gonflable souple, ladite ceinture étant rapportée contre la bande gonflable souple et fixée à cette dernière.

### TECHNIQUE ANTERIEURE

Il est connu d'avoir recours à des dispositifs implantables pour réduire le volume de l'estomac ou le diamètre de son stoma de manière à faciliter la perte de poids d'un patient dans le cadre d'un traitement de l'obésité morbide. En effet, lorsque les méthodes traditionnelles de régime alimentaire s'avèrent inefficaces et/ou lorsque l'obésité du patient s'avère particulièrement dangereuse pour sa santé (problèmes cardio-vasculaires, articulaires, diabète, ...), il est préférable d'avoir recours à des méthodes chirurgicales consistant en l'implantation de dispositifs destinés à réduire l'apport alimentaire.

Parmi les méthodes connues, il est possible d'avoir recours à une constriction gastrique à l'aide d'un anneau de gastroplastie destiné à être implanté autour de l'estomac, au cours d'une intervention chirurgicale, de manière à réduire sensiblement le diamètre de passage des aliments dans l'estomac.

On connaît ainsi un anneau de gastroplastie comportant d'une part une bande dorsale formant ceinture et d'autre part une bande gonflable souple rapportée et fixée contre la ceinture, le long de cette dernière, pour former un anneau gastrique unitaire. La fermeture de l'anneau est assurée par un dispositif de verrouillage approprié positionné aux extrémités de la ceinture et permettant à l'anneau d'enserrer l'estomac de manière fonctionnelle, selon une configuration sensiblement circulaire.

La principale fonction de la bande dorsale est de contenir la déformation de la bande gonflable souple de manière à favoriser un gonflage majoritairement centripète de cette dernière, vers l'estomac.

Le volume de la bande gonflable est réglé par ajout ou retrait d'un fluide de gonflage. A cette fin, l'anneau connu évoqué ci-avant est pourvu d'un cathéter relié à l'une de ses extrémités à la bande gonflable souple de l'anneau et à l'autre extrémité à un site implantable sous-cutané permettant l'introduction ou le retrait de fluide de gonflage à travers la peau du patient à l'aide d'une aiguille creuse transperçant la membrane étanche du site implantable.

Un tel anneau présente un certain nombre d'inconvénients.

Ainsi, dans cet anneau connu, le cathéter fait saillie à partir de la bande gonflable souple, près de l'estomac, ce qui est susceptible de gêner le chirurgien lors de la manipulation de l'anneau pour sa mise en place autour de l'estomac. En effet, le chirurgien peut être amené à prendre des précautions particulières afin de dégager parfaitement le cathéter de l'anneau, pour éviter notamment de le coincer entre la bande gonflable souple et l'estomac.

En outre, l'arrivée du cathéter sur la bande gonflable souple à proximité de l'estomac favorise le frottement du cathéter avec l'estomac voire sa retenue dans des plis éventuels de l'estomac, ce qui peut provoquer des nécroses de la paroi externe de l'estomac et rendre l'anneau particulièrement inconfortable pour le patient.

De surcroît, ce type d'anneau prévoit un assemblage du cathéter à la bande gonflable souple par un simple point de fixation, susceptible d'être fortement sollicité par le frottement du cathéter avec l'estomac et les tissus environnants. Un tel frottement peut provoquer une fragilisation de la fixation du cathéter voire entraîner sa détérioration potentielle et un possible débranchement intempestif du cathéter.

Une telle fixation du cathéter sur la bande gonflable peut également gêner le gonflage homogène de l'anneau, en pouvant par exemple favoriser la formation de plis éventuels dans la bande gonflable souple. Ce type de fixation du cathéter peut donc ne pas s'avérer un moyen fiable assurant un gonflage simple et sûr de l'anneau.

Aux difficultés de fonctionnement précédemment évoquées peuvent également venir s'ajouter d'éventuels problèmes de la fabrication. En effet, la fabrication d'un tel anneau s'avère particulièrement compliquée, notamment pour l'assemblage du cathéter sur la bande gonflable. En général, la bande gonflable est réalisée avec un matériau élastomère présentant une rigidité plus faible que celle du cathéter, ce qui entraîne une opération d'assemblage particulièrement délicate et longue, au cours de laquelle l'intervention humaine peut s'avérer nécessaire, pour parvenir à un maintien stable et étanche du cathéter sur la bande gonflable. Cette étape alourdit sensiblement le procédé de fabrication et contribue à le rendre onéreux.

Bien entendu, de tels inconvénients peuvent nécessiter de nouvelles opérations chirurgicales, par exemple pour retirer un anneau détérioré, qui mobilisent inutilement du personnel médical, alourdissent le traitement, causent des désagréments supplémentaires au patient, voire exposent ce dernier à des complications postopératoires.

Un anneau chirurgical selon le préambule de la revendication 1 est connu du document EP-A-1 829 504.

### EXPOSE DE L'INVENTION

Les objets assignés à la présente invention visent par conséquent à porter remède aux inconvénients susmentionnés et à proposer un nouvel anneau chirurgical implantable facile à mettre en oeuvre pour le chirurgien, de caractère atraumatique, de construction robuste, facile et peu onéreux à fabriquer industriellement.

Un autre objet assigné à la présente invention vise à proposer un nouvel anneau chirurgical implantable présentant un fonctionnement particulièrement simple et facile à mettre en oeuvre.

Un autre objet assigné à la présente invention vise à proposer un nouvel anneau chirurgical implantable dont la mise en oeuvre est rapide et simple.

Un autre objet assigné à la présente invention vise à proposer un nouvel anneau chirurgical implantable garantissant la sécurité et l'étanchéité du gonflage.

Un autre objet assigné à la présente invention vise à proposer un nouvel anneau chirurgical implantable présentant un maintien stable et durable en position fonctionnelle.

Un autre objet assigné à la présente invention vise à proposer un nouvel anneau chirurgical implantable qui présente une configuration atraumatique et particulièrement respectueuse des tissus environnants.

Un autre objet assigné à la présente invention vise à proposer un nouvel anneau chirurgical implantable de conception simple et dont l'assemblage des parties constitutives est facilité.

Un autre objet assigné à la présente invention vise à proposer un nouvel anneau chirurgical implantable permettant un gonflage simple et fonctionnel sans fuite de fluide de gonflage.

Un autre objet assigné à la présente invention vise à proposer un nouvel anneau chirurgical implantable présentant un gonflage homogène limitant les plis.

Un autre objet assigné à la présente invention vise à proposer un nouvel anneau chirurgical implantable qui présente une structure simple, légère, facile à mettre en oeuvre et peu onéreuse.

Un autre objet assigné à la présente invention vise à proposer un nouvel anneau chirurgical implantable utilisé pour le traitement de l'obésité.

Un autre objet assigné à l'invention vise à proposer un nouveau procédé de fabrication d'un anneau chirurgical implantable, facile et peu onéreux à réaliser industriellement, qui confère audit anneau une mise en oeuvre facile pour le chirurgien, un caractère atraumatique, une construction robuste.

Un autre objet assigné à l'invention vise à proposer un nouveau procédé de fabrication d'un anneau chirurgical implantable qui facilite l'assemblage et le maintien stable dudit anneau en position fonctionnelle.

Un autre objet assigné à l'invention vise à proposer un nouveau procédé de fabrication d'un anneau chirurgical implantable qui soit simple, peu onéreux et rapide à mettre en oeuvre.

Un autre objet assigné à l'invention vise à proposer un nouveau procédé de fabrication d'un anneau chirurgical implantable conférant à l'anneau une excellente stabilité en position fonctionnelle.

Enfin un dernier objet assigné à l'invention vise à proposer un nouveau procédé de fabrication d'un anneau chirurgical implantable permettant d'obtenir un anneau d'utilisation gastrique.

Les objets assignés à l'invention sont atteints à l'aide d'un anneau chirurgical implantable selon la revendication 1.

Les objets assignés à l'invention sont également atteints à l'aide d'un procédé de fabrication d'un anneau chirurgical implantable tel que défini à la revendication 10.

### DESCRIPTIF SOMMAIRE DES DESSINS

D'autres objets, caractéristiques et avantages de l'invention apparaîtront plus en détails à la lecture de la description qui suit, ainsi qu'à l'aide des dessins annexés, donnés à titre purement illustratif et non limitatif, parmi lesquels :

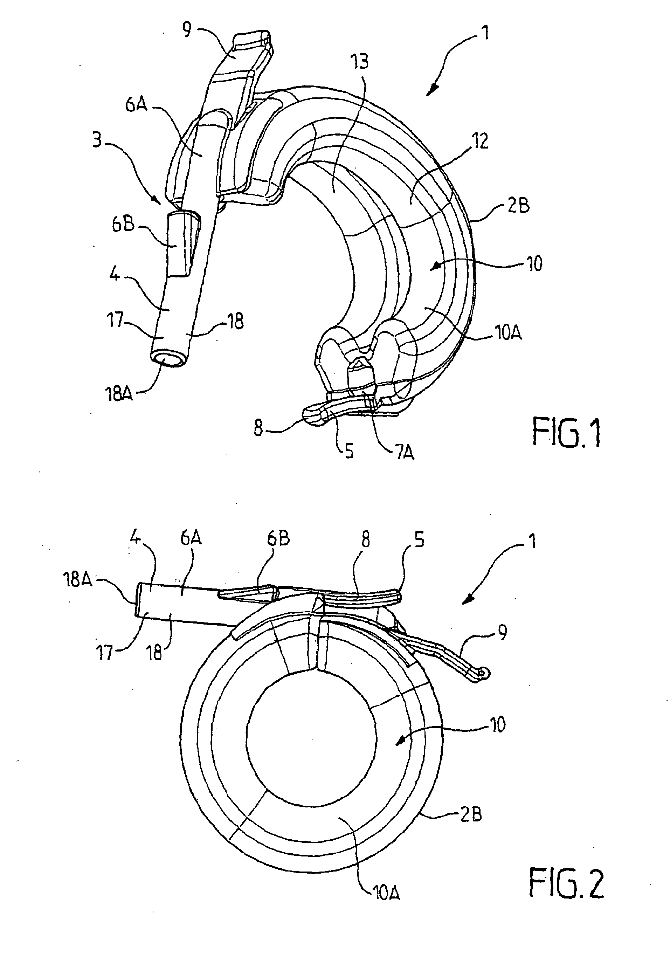

- La figure 1 illustre, selon une vue en perspective de profil, un anneau chirurgical conforme à l'invention dans sa position ouverte.
- La figure 2 illustre, selon une vue de côté, un anneau chirurgical conforme à l'invention dans sa position fermée.
- La figure 3 illustre, selon une vue éclatée en perspective de profil, l'anneau chirurgical de la figure 1 selon un premier mode de réalisation.
- La figure 4 illustre, selon une vue éclatée en perspective de profil, l'anneau chirurgical de la figure 1 selon un deuxième mode de réalisation.

### MEILLEURE MANIERE DE REALISER L'INVENTION

Dans la description qui suit, nous nous attacherons à décrire, à titre d'exemple et par souci de commodité, un anneau gastrique (ou anneau de gastroplastie) destiné à venir entourer l'estomac en vue de réduire la section de passage de ce dernier, dans le cadre d'un traitement de l'obésité.

La présente invention n'est cependant pas limitée à une application gastrique de l'anneau, et couvre également de manière générale les anneaux chirurgicaux destinés à être implantés dans le corps d'un patient autour d'un organe biologique constituant une poche ou un conduit en vue de modifier la section de passage dudit organe lorsque ce dernier est enserré par l'anneau.

Ainsi, la présente invention peut être par exemple utilisée dans le cadre d'un traitement de l'incontinence urinaire ou fécale, ou encore pour la régulation du débit sanguin.

En fonction de l'utilisation qui en sera faite, le présent anneau pourra présenter des dimensions, une forme et des fonctionnalités adaptées à l'organe concerné par la constriction, comme par exemple la vessie, l'urètre, l'intestin, une artère, une veine, etc.

La présente invention concerne donc un anneau chirurgical 1 implantable dans un corps humain ou animal, de préférence un anneau gastrique 1. Ledit anneau 1 est destiné à venir entourer un organe biologique constituant une poche ou un conduit, notamment l'estomac du patient, en vue de modifier la section de passage dudit organe, en particulier pour réduire le stoma de l'estomac et limiter ainsi la quantité d'aliments ingérés par le patient.

L'anneau 1 est réalisé à partir de plusieurs pièces constitutives assemblées pour former un anneau 1 fonctionnel unitaire. L'anneau 1 comprend d'une part une ceinture 2, conçue pour former la partie la plus extérieure de l'anneau 1, savoir la partie la plus éloignée de l'estomac en contact avec les tissus environnants. La ceinture 2 est réalisée en matériau élastomère, préférentiellement en silicone, et présente avantageusement une mémoire de forme circulaire. Ainsi, la ceinture 2 se présente sous une forme sensiblement arrondie en position ouverte (au repos), ce qui favorise bien évidemment sa mise en forme autour de l'estomac.

La ceinture 2 comprend une face interne 2A définissant le périmètre intérieur de la ceinture 2, c'est-à-dire le plus proche de l'estomac, ainsi qu'une face externe 2B, définissant le périmètre extérieur de la ceinture, c'est-à-dire celui le plus éloigné de l'estomac et destiné à venir en contact avec les tissus environnants.

La ceinture 2 comporte au moins un moyen de verrouillage 3 dudit anneau en position fonctionnelle fermée autour dudit organe biologique. Le moyen de verrouillage 3 comprend au moins deux éléments de fermeture 6, 7 conçus pour coopérer entre eux afin d'assurer le verrouillage de l'anneau 1 dans sa configuration fermée autour de l'estomac, les éléments de fermeture 6 et 7 étant positionnés de préférence au niveau des extrémités 4 et 5 respectivement de la ceinture 2.

L'élément de fermeture 7 présente avantageusement une forme de manchon 7A destiné à accueillir l'élément de fermeture 6 formant tige 6A. Ces deux éléments de fermeture 6, 7 sont destinés à être emboités l'un dans l'autre de manière stable, grâce notamment à la présence d'au moins un ergot 6B présent sur la tige 6A. En effet, l'ergot 6B présente préférentiellement une face d'introduction favorisant le passage par déformation élastique de la tige 6A dans le manchon 7A et une face de non-retour permettant d'éviter l'ouverture intempestive de l'anneau 1 une fois la tige 6A introduite dans le manchon 7A au-delà de l'ergot 6B.

En position fermée, lorsque les éléments de fermeture 6, 7 du moyen de verrouillage 3 coopèrent l'un dans l'autre, la ceinture 2 présente une forme sensiblement arrondie, préférentiellement sensiblement circulaire.

Par ailleurs, la ceinture 2 de l'anneau peut être pourvue d'une ou plusieurs languettes de verrouillage/déverrouillage 8 destinées à faciliter la manipulation par coelioscopie de l'anneau 1, notamment lors de sa fermeture et/ou de son ouverture. En effet, la languette de verrouillage/déverrouillage 8 est préférentiellement formée par l'extrémité 5 de la ceinture 2 et peut être utilisée par le chirurgien à l'aide d'un instrument de préhension pour permettre de fermer et/ou d'ouvrir l'anneau 1 en tirant dessus.

De manière avantageuse, la ceinture 2 comporte, en outre, une ou plusieurs languettes de préhension 9 destinée(s) également à faciliter l'utilisation de l'anneau 1 dans le corps du patient. En effet, grâce à une telle languette de préhension 9, le chirurgien peut facilement manipuler l'anneau, notamment au moment de sa fermeture et/ou de son ouverture.

L'anneau 1 comprend d'autre part une bande gonflable souple 10 comportant au moins une ouverture 11 permettant l'apport en son sein d'un fluide de gonflage. De préférence, la bande gonflable souple 10 est indépendante de la ceinture 3 et forme une poche, c'est-à-dire une chambre fermée et individualisée. La bande gonflable 10 correspond donc avantageusement à un boyau souple formant poche étanche au fluide de gonflage.

De manière avantageuse, le fluide de gonflage est un gaz ou un liquide, de préférence du sérum physiologique. La bande gonflable souple 10 est de préférence réalisée en matériau élastomère, avantageusement en silicone, avec un mémoire de forme sensiblement circulaire.

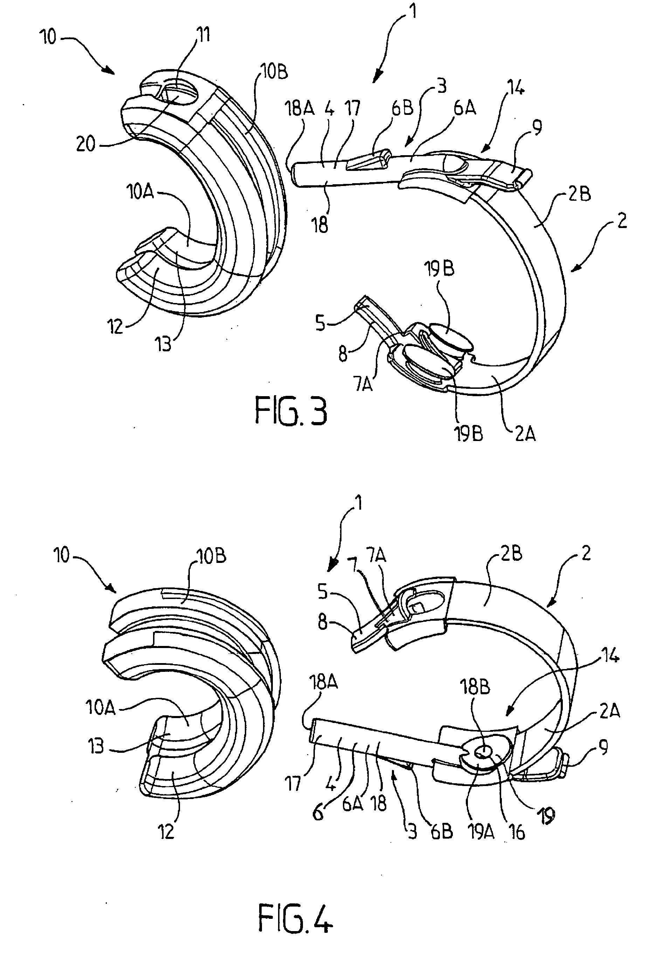

La bande gonflable souple 10 comprend une face interne 10A en contact avec l'estomac et une face externe 10B opposée. La bande gonflable souple 10 comprend également au moins une chambre de serrage annulaire 12 destinée à recevoir le fluide de gonflage. La chambre 12 permet donc de manière avantageuse de réaliser un enserrement sensiblement en forme d'anneau autour de l'organe, de préférence de l'estomac. De préférence, la bande gonflable souple 10 comprend au moins deux chambres de serrage annulaire 12, 13 qui s'étendent sensiblement parallèlement l'une à l'autre, tel qu'on peut le voir aux figures 3 et 4. Lesdites chambres 12, 13, qui sont avantageusement étagées l'une par rapport à l'autre, reçoivent le fluide de gonflage par l'ouverture 11 ménagée avantageusement sur la face externe 10B de la bande gonflable souple 10.

L'apport de fluide de gonflage dans la bande gonflable souple 10 permet de modifier le volume des chambres de serrage annulaire 12, 13, de sorte à permettre l'enserrage efficace de l'estomac.

Sans sortir du cadre de cette invention, il est également envisageable que la bande gonflable souple 10 comprenne, sur sa face externe 10B, plusieurs ouvertures 11 permettant l'arrivée du fluide de gonflage.

La ceinture 2 est rapportée contre la bande gonflable souple 8, à l'extérieur de cette dernière, et fixée à la bande gonflable souple 8. En effet, tel que cela est illustré aux figures, la face interne 2A de la ceinture 2 est positionnée sur la face externe 10B de la bande gonflable souple 10. En d'autres termes, la bande gonflable souple 10 et la ceinture 2, qui sont parfaitement distinctes, séparées, individualisées et indépendantes l'une de l'autre, sont assemblées au niveau et sur toute la longueur de leur face externe 10B et interne 2A respectivement. De préférence, la fixation de la bande gonflable souple 10 contre et le long de la ceinture 2 est uniforme, de telle sorte que la bande souple gonflable 10 et la ceinture 2 sont substantiellement coextensives.

De manière avantageuse, la face interne 2A de la ceinture 2 présente une forme sensiblement complémentaire de celle de la face externe 10B de la bande gonflable souple 10 contre laquelle ladite ceinture 2 est rapportée. De préférence, la face interne 2A de la ceinture 2 comprend sur toute sa longueur une bande surélevée (non représentée), avantageusement de la forme d'un parallélépipède rectangle, destinée à venir s'emboîter dans une bande creuse présente sur toute la longueur de la face externe 10B de la bande gonflable souple 10 et de forme sensiblement complémentaire de celle de la bande surélevée de la ceinture 2, c'est-à-dire de la forme d'un parallélépipède rectangle creux. Une telle complémentarité de forme entre les faces 2A et 10B favorise l'assemblage stable de la ceinture 2 avec la bande gonflable souple 10.

La ceinture 2 fixée sur la bande 10 permet de favoriser un gonflage centripète de cette dernière, une fois l'anneau implanté autour de l'estomac, de manière à enserrer efficacement ce dernier. En effet, la ceinture 2 joue le rôle de dorsale pour la bande gonflable souple 10. D'ailleurs, l'assemblage de ladite ceinture 2 à l'extérieur de la bande gonflable souple 10 pour réaliser l'anneau 1 présente l'avantage de permettre l'utilisation de matériaux de nature différente pour chacune desdites ceinture 2 et bande 10. Dans un mode de réalisation particulièrement avantageux, la ceinture 2 est réalisée dans un matériau élastomère présentant une rigidité supérieure et une élasticité inférieure à celle de l'élastomère constitutif de la bande 10, de manière à favoriser le gonflage centripète de l'anneau lors de l'apport du fluide de gonflage dans la bande 10.

La ceinture 2 de la présente invention comprend un moyen de mise en communication 14 de ladite au moins une ouverture 11 de la bande gonflable souple 10 avec un moyen d'apport de fluide de gonflage 15. Avantageusement, le fluide de gonflage est amené au niveau de la ceinture 2 qui en assure ensuite le transit jusqu'à la bande gonflable souple 10 grâce à un moyen de mise en communication 14. La ceinture 2 joue donc un rôle d'interface pour la transmission du fluide de gonflage du site implantable vers la bande gonflable souple 10. En d'autres termes, le fluide de gonflage transite avantageusement à travers la ceinture 2 avant de rejoindre la bande gonflable souple 10.

Dans un mode de réalisation préférentiel de la présente invention, le moyen d'apport de fluide de gonflage 15 (non représenté aux figures) comprend un conduit, de préférence un cathéter. En général, le cathéter est relié à un site implantable sous-cutané permettant l'apport et/ou le retrait de fluide de gonflage à l'aide d'une aiguille insérée dans le site et reliée à une seringue. En d'autres termes, le cathéter est connecté sur la ceinture 2 et le transfert du fluide entre le cathéter et la ceinture 2 se fait au niveau du moyen de mise en communication 14. Le fluide de gonflage transite donc par la ceinture 2 avant d'atteindre la bande gonflable souple 10.

De manière avantageuse, ce moyen de mise en communication 14 comprend un orifice 16, ménagé dans la ceinture, qui assure la communication fluidique avec ladite ouverture 11 de la bande gonflable souple 10. De préférence, l'orifice 16 de la ceinture 2 est rapporté contre l'ouverture 11 de la bande gonflable souple 10 de manière à établir une communication fluidique étanche entre ladite bande 10 et ladite ceinture 2. En d'autres termes, l'orifice 16 ménagé dans la ceinture 2, de préférence au niveau de sa face interne 2A, est abouché à l'ouverture 11, de manière à établir une communication fluidique entre la ceinture 2 et la bande 10.

De manière avantageuse, l'orifice 16 de la ceinture 2 permet également la communication fluidique entre la bande gonflable souple 2, qui comprend au moins deux chambres de serrage annulaire 12, 13, et le moyen d'apport de fluide de gonflage 15. Cette communication fluidique s'établit à l'aide du moyen de mise en communication 14 de la ceinture 2 qui d'une part reçoit le cathéter amenant le fluide de gonflage et d'autre part transfère le fluide de gonflage à la bande de gonflage souple 10, par l'orifice 16 rapporté contre l'ouverture 11 de ladite bande 10. Le fluide de gonflage est ainsi avantageusement transféré via la ceinture 2 dans la bande gonflable 10 et traverse donc ladite ceinture 2.

De préférence, l'orifice 16 de la ceinture 2 assure également la communication fluidique entre chacune des chambres de serrage 12, 13. En effet, la mise en contact étanche de l'orifice 16 de la ceinture 2 avec l'ouverture 11 de la bande gonflable souple 10 permet avantageusement la création d'une zone de transfert de fluide 20, permettant à la fois le remplissage de chacune des chambres de serrage annulaire 12, 13 et la communication fluidique entre ces deux chambres 12, 13. Cette zone 20 présente l'intérêt de garantir d'une part un gonflage homogène de l'anneau 1 après son implantation autour de l'estomac et d'autre part son dégonflage harmonieux en fin d'utilisation. En outre, la zone 20 favorise l'équilibrage du volume des deux chambres 12, 13 au cours de l'utilisation en permettant le transfert de fluide d'une chambre à l'autre.

Alternativement, dans un autre mode de réalisation, il est également envisageable que les deux chambres 12, 13 soient complètement séparées fluidiquement, c'est-à-dire qu'aucun transfert de fluide n'est possible entre lesdites chambres 12, 13, à l'aide par exemple d'une cloison intermédiaire séparant les chambres 12, 13 sur toute leur longueur. Dans ce cas, chaque chambre 12, 13 dispose d'un orifice 16 par lequel est apporté et/ou retiré le fluide de gonflage. Le transfert de fluide de gonflage peut se faire à l'aide un cathéter unique relié aux deux chambres 12, 13 ou à l'aide de deux cathéters reliés chacun à une chambre et permettant une gestion indépendante de chacune desdites chambres 12, 13.

De préférence, le moyen de mise en communication 14 comprend un conduit 17 qui est connecté d'une part à l'orifice 16 et d'autre part au moyen d'apport de fluide de gonflage 15, c'est-à-dire au cathéter. Avantageusement, le conduit 17 est sensiblement confondu avec la tige 7 formant l'élément de fermeture du moyen de verrouillage 3. Le fluide de gonflage est en effet amené par le cathéter au conduit 17 et transmis ensuite par ledit conduit 17 à la bande gonflable souple 10 à travers l'orifice 16, ménagé à l'extrémité du conduit 17 sur la ceinture 2. Ce conduit 17, faisant saillie à partir de l'anneau 1 lors de sa fermeture autour de l'estomac, tel qu'illustré à la figure 3, facilite ainsi la manipulation de l'anneau 1 par le chirurgien et évite notamment que le cathéter reste contre l'estomac et/ou gêne le chirurgien lors de la manipulation de l'anneau 1.

Dans un mode de réalisation préférentiel, le moyen de mise en communication 14 forme un logement 18 qui entoure au moins une partie dudit moyen d'apport de fluide de gonflage 15, à savoir le cathéter. Tel qu'on peut le voir aux figures, le logement 18 est conçu pour recevoir au niveau de sa première extrémité 18A le cathéter (non représenté), inséré et fixé stablement dans le logement 18, et pour déboucher au niveau de sa deuxième extrémité 18B sur l'orifice 16 rapporté contre l'ouverture 11 de la bande 10. Le logement 18 permet donc le transfert étanche de fluide de gonflage du cathéter vers la bande 10.

Avantageusement, le logement 18, par sa forme en étui et ses caractéristiques de souplesse liées notamment au matériau qui le constitue, épouse parfaitement le cathéter qui est inséré, de préférence en force, en son sein. Afin d'assurer la fixation solide et durable du cathéter dans le logement 18, on procède généralement au collage du cathéter, de préférence par enduction de la surface extérieure de la partie du cathéter insérée dans le logement à l'aide d'une colle ou d'un matériau adhésif, de préférence biocompatible. De manière alternative, il est également envisageable de surmouler le cathéter dans le logement 18 de la ceinture 2.

Le moyen de mise en communication 14, notamment le logement 18, garantit donc une fixation sûre du cathéter sur la ceinture 2. De plus, le logement 18, réalisé en matériau élastomère, de type silicone, présente une certaine rigidité par rapport au cathéter permettant de dégager ce dernier de l'estomac et ainsi éviter les frottements du cathéter avec l'estomac.

Ce moyen de mise en communication 14 permet également de favoriser l'assemblage de la ceinture 2 avec la bande 10. En effet, le moyen de mise en communication 14 comprend un moyen de fixation 19 conçu pour coopérer avec l'ouverture 11 de la bande gonflable souple 10 afin de contribuer, au moins vocalement, à la solidarisation de la ceinture 2 à la bande gonflable souple 10.

Dans un mode de réalisation préférentiel, le moyen de fixation 19 se présente sous la forme d'un bouton 19A dont la configuration est sensiblement complémentaire de celle de l'ouverture 11 de la bande gonflable souple 10, ladite ouverture 11 formant une boutonnière conçue pour accueillir ledit moyen de fixation 19. Tel qu'illustré à la figure 4, le bouton 19A de la ceinture 2 est avantageusement percé par l'orifice 16 et inséré dans l'ouverture 11 de la bande 10, une telle configuration permettant d'assurer d'une part l'assemblage, au moins local, de la ceinture 2 avec la bande 10, et d'autre part la communication fluidique entre ladite ceinture 2 et ladite bande 10.

La solidarisation de la ceinture 2 avec la bande 10 au niveau du moyen de fixation 19, à la manière d'un boutonnage ou d'un encliquetage, permet avantageusement une tenue relative de la ceinture 2 sur la bande 10 de manière à faciliter leur fixation ultérieure par collage.

Il est également envisageable, sans pour autant sortir du cadre de la présente invention, que le moyen de fixation 19 comprenne plusieurs boutons 19A, 19B positionnés préférentiellement au niveau de chacune des extrémités 4, 5 respectivement de la ceinture 2. Le bouton 19A comprenant l'orifice 16 est inséré dans l'ouverture 11 de la bande 10 tandis que le ou les boutons 19B non percé(s) d'un orifice sont insérés dans deux ouvertures (non représentées) réalisées sur la face interne 10A de chacune des chambres de serrage annulaires 12, 13 de la bande 10. Lesdits au moins trois boutons 19A, 19B, tels qu'illustrés à la figure 3, facilitent la tenue stable de la ceinture 2 sur la bande gonflable souple 10 en vue de leur fixation ultérieure, par exemple par collage, tout en permettant, grâce au bouton 19A comprenant l'orifice 16, l'établissement d'une communication fluidique simple et étanche entre la ceinture 2 et la bande 10.

Au vu de ce qui précède, la ceinture 2 de l'anneau 1 de la présente invention comprend donc avantageusement le moyen de verrouillage 3, la patte de préhension 9 et le moyen de mise en communication 14, comprenant lui-même l'orifice 16, le conduit 17, le logement 18 et le moyen de fixation 19.

De préférence, la ceinture 2 est formée d'une pièce d'un seul tenant comprenant d'une part sur sa face interne 2A l'orifice 16 et d'autre part dans le prolongement de l'une de ses extrémités 4 le conduit 17, de manière à former une ceinture 2 monobloc. De manière avantageuse, la ceinture 2 est une pièce unique comportant notamment le moyen de mise en communication 14, ce qui lui confère une configuration monobloc simple et fonctionnelle, particulièrement atraumatique pour le patient.

En effet, la fabrication en une seule pièce de l'ensemble des éléments constitutifs de ladite ceinture 2 permet d'éviter les zones de jonction pouvant devenir abrasives pour l'estomac et les tissus. En outre, une telle structure monobloc évite le détachement intempestif et potentiellement dangereux de certains éléments de la ceinture 2 qui pourraient se déplacer librement dans le corps humain.

Dans un mode de réalisation avantageux de l'invention, la bande gonflable souple 10 est également formée d'une pièce d'un seul tenant de manière à former une bande gonflable souple 10 monobloc, indépendante et séparée de ladite ceinture 2. A l'image de la ceinture 2 monobloc, la bande gonflable souple 10 formant boyau individuel fermé est formée d'une seule pièce comprenant les éléments constitutifs précédemment cités, comme par exemple, l'ouverture 11 et/ou la bande creuse sur la face externe 10B et/ou les chambres de serrage annulaire 12, 13.

Selon la présente invention, la configuration de l'anneau 1 selon laquelle la ceinture 2 monobloc est rapportée contre et le long de la bande gonflable souple 10, à l'extérieur de la bande 10, et fixée à cette dernière, est particulièrement atraumatique et facile à mettre en oeuvre.

D'un point de vue fonctionnel, l'anneau 1, qui comprend la bande gonflable souple 10, la ceinture 2 sur laquelle est branché le cathéter au niveau du logement 18, est introduit dans le corps du patient et est positionné de manière à entourer l'estomac de ce dernier. Le chirurgien ferme l'anneau 1 autour de l'estomac en introduisant le cathéter et le logement 18 dans le manchon 7A de la ceinture 2 jusqu'à dépasser l'ergot 6A permettant le verrouillage de l'anneau 1, de forme sensiblement circulaire, dans sa position fonctionnelle. Le cathéter est ensuite relié à un site implantable sous-cutané à travers lequel le chirurgien apporte le fluide de gonflage à l'aide d'une aiguille reliée à une seringue. Ce fluide transite dans le cathéter, arrive au logement 18 de la ceinture 2, traverse l'orifice 16 de la ceinture 2 et arrive dans la bande gonflable souple 10 au niveau de son ouverture 11 communiquant avec l'orifice 16, le fluide de gonflage transitant donc avantageusement à travers la ceinture 2 avant d'atteindre la bande gonflable 10. La bande gonflable souple 10 se gonfle alors de manière centripète de manière à enserrer l'estomac pour en réduire le stoma.

La présente invention concerne également un procédé de fabrication d'un anneau chirurgical implantable dans un corps humain ou animal, ledit anneau étant destiné à venir entourer un organe biologique constituant une poche ou un conduit en vue de modifier la section de passage dudit organe. De préférence, la présente invention concerne un procédé de fabrication d'un anneau gastrique 1, tel que décrit dans ce qui précède, destiné à venir entourer l'estomac en vue de réduire la section de passage de ce dernier dans le cadre d'un traitement de l'obésité. La suite de la description s'attachera donc à décrire un procédé de fabrication d'un anneau gastrique tel que défini dans ce qui précède.

Le procédé comprend une étape de fabrication d'une ceinture 2 comportant au moins un moyen de verrouillage 3 dudit anneau 1 en position fonctionnelle fermée autour dudit organe biologique.

Ce procédé comprend en outre une étape de fabrication d'une bande gonflable souple 10 comprenant au moins une chambre de serrage annulaire 12 destinée à recevoir un fluide de gonflage et comportant au moins une ouverture 11 permettant l'apport en son sein du fluide de gonflage, ladite bande souple 10 formant un boyau fermé indépendant, distinct et séparé de ladite ceinture 2.

Après la fabrication de la ceinture 2 et de la bande 10, le procédé de l'invention comprend une étape d'assemblage de la ceinture 2 avec la bande gonflable souple 10, ladite ceinture 2 étant rapportée contre la bande gonflable souple 10 et fixée à cette dernière. Avantageusement, l'opération de fixation de la bande gonflable souple 10 contre et le long de la ceinture 2 se fait de manière uniforme sur toute la longueur desdites bande 10 et ceinture 2, de manière à ce que ces dernières soient sensiblement coextensives.

Dans un mode de réalisation préféré, l'étape de fabrication de la ceinture 2 comprend une sous-étape de fabrication de la face interne 2A de ladite ceinture 2, ladite face interne 2A présentant une forme sensiblement complémentaire de celle de la face externe 10B de la bande gonflable souple 10 contre laquelle ladite face interne 2A de la ceinture 2 est rapportée, ladite face externe 10B étant fabriquée au cours de l'étape de fabrication de ladite bande gonflable 10. De manière avantageuse, on réalise sur la face interne 2A de la ceinture 2 une bande surélevée, de la forme d'un parallélépipède rectangle et de forme sensiblement identique à celle d'une bande creuse réalisée sur la face externe 10B de la bande 10, de manière à permettre un emboîtement stable par complémentarité de forme de la face interne 2A de la ceinture 2 sur la face externe 10B de la bande 10.

Préférentiellement, cet assemblage stable de la ceinture 2 avec la bande gonflable souple 10 met par ailleurs en oeuvre une opération de collage au cours de laquelle la face externe 10B de la bande gonflable 10, de préférence les bords extérieurs entourant la bande creuse susmentionnée, et/ou la face interne 2A de la ceinture 2, de préférence les bords extérieurs entourant la bande surélevée précitée, reçoit une quantité de colle suffisante pour garantir un collage efficace de la bande 10 avec la ceinture 2.

L'étape de fabrication de la ceinture 2 dudit procédé de fabrication comprend également une sous-étape de fabrication d'un moyen de mise en communication 14 de ladite au moins une ouverture 11 de la bande gonflable souple 10 avec un moyen d'apport de fluide de gonflage 15, de préférence un cathéter tel que décrit dans ce qui précède.

Avantageusement, la sous-étape de fabrication du moyen de mise en communication 14 comprend une opération de fabrication d'un dispositif de fixation 19, présentant une forme sensiblement complémentaire de celle de l'ouverture 11 de la bande gonflable souple 10, ledit dispositif de fixation 19 étant destiné à assurer un assemblage stable de ladite ceinture 2 avec ladite bande gonflable souple 10 pour former l'anneau 1.

De préférence, l'étape de fabrication de la bande gonflable souple 10 comprend une sous-étape de fabrication d'au moins deux chambres de serrage annulaire 12, 13 qui s'étendent sensiblement parallèlement l'une à l'autre, la mise en communication fluidique entre la bande gonflable souple 10 et le moyen d'apport de fluide de gonflage 14 étant assurée par l'orifice 16 de la ceinture 2.

Par ailleurs, l'étape de fabrication de la ceinture 2 se fait, de manière avantageuse, par moulage d'une pièce d'un seul tenant de manière à former une ceinture 2 monobloc comprenant d'une part sur sa face interne 2A un orifice 16 permettant la communication fluidique avec ladite ouverture 11 de la bande gonflable souple 10 et comprenant d'autre part dans son prolongement le moyen de mise en communication 14 formant un conduit 17, ledit conduit 17 étant connecté d'une part à l'orifice 16 et d'autre part au moyen d'apport de fluide de gonflage 15, c'est-à-dire au cathéter. De préférence, le moulage de la ceinture 2 peut également comprendre le surmoulage du cathéter dans le conduit 17, de manière à permettre un assemblage sûr et étanche du cathéter avec la ceinture 2.

L'étape de moulage fait intervenir de préférence un moule en deux parties dans lequel est réalisée une empreinte précise de ladite ceinture 2 avec ses différents éléments constitutifs (le conduit 17, logement 18, moyen de fixation 19, orifice 16, moyen de verrouillage 3, etc.). Le moule est ensuite rempli avec le matériau constitutif de la ceinture 2 sous une forme liquide, de préférence du silicone, et refermé. Après refroidissement et démoulage, ce procédé permet d'obtenir une ceinture monobloc comprenant l'ensemble des éléments précités.

Le moulage monobloc de la ceinture 2 est particulièrement avantageux en termes de rapidité, de coût et de simplicité de réalisation. Il permet de limiter le nombre d'étapes nécessaire à la fabrication de ladite ceinture 2 et assure une configuration atraumatique de cette dernière.

En outre, il est également envisageable que l'étape de fabrication de la bande gonflable souple 10 se fasse avantageusement par moulage d'une pièce d'un seul tenant de manière à former une bande gonflable souple 10 monobloc. Il est bien évidemment tout à fait envisageable sans sortir du cadre de la présente invention, de fabriquer la ceinture 2 monobloc et/ou la bande gonflable souple 10 monobloc par une méthode autre que le moulage.

La fabrication monobloc de la ceinture 2, et préférentiellement de la bande gonflable souple 2, limite sensiblement le nombre d'étapes du procédé de fabrication de l'anneau 1, ce qui réduit considérablement le temps et les coûts de fabrication de l'anneau 1.

### POSSIBILITE D'APPLICATION INDUSTRIELLE

L'invention trouve son application industrielle dans la conception et la fabrication de dispositifs médicaux implantables notamment destinés au traitement de l'obésité.

## Revendications

1. Anneau chirurgical implantable (1) dans un corps humain ou animal, ledit anneau (1) étant destiné à venir entourer un organe biologique constituant une poche ou un conduit en vue de modifier la section de passage dudit organe, ledit anneau (1) comprenant:
d'une part une ceinture (2) comportant au moins un moyen de verrouillage (3) dudit anneau (1) en position fonctionnelle fermée autour dudit organe biologique, comprenant un moyen de mise en communication (14) de ladite au moins une ouverture (11) de la bande gonflable souple (10) avec un moyen d'apport de fluide de gonflage et
d'autre part une bande gonflable souple (10) comprenant au moins une chambre de serrage annulaire (12) destinée à recevoir un fluide de gonflage et comportant au moins ladite ouverture (11) permettant l'apport en son sein du fluide de gonflage, ladite ceinture (2) étant rapportée contre la bande gonflable souple (10) et fixée à cette dernière;
-ledit anneau (1) étant **caractérisé en ce que** ledit moyen de mise en communication (14) comprend un moyen de fixation (19) conçu pour coopérer avec l'ouverture (11) de la bande gonflable souple (10) afin de contribuer, au moins localement, à la solidarisation de la ceinture (2) à la bande gonflable souple (10), le moyen de fixation (19) se présentant sous la forme d'un bouton (19A) dont la configuration est sensiblement complémentaire de celle de l'ouverture (11) de la bande gonflable souple (10), ladite ouverture (11) formant une boutonnière conçue pour accueillir ledit moyen de fixation (19).

2. Anneau chirurgical (1) selon la revendication 1 **caractérisé en ce que** le moyen de mise en communication (14) comprend un orifice (16) qui assure la communication fluidique avec ladite ouverture (11) de la bande gonflable souple (10).

3. Anneau chirurgical (1) selon la revendication 1 ou 2 **caractérisé en ce que** le moyen de mise en communication (14) comprend un conduit (17) qui est connecté d'une part à l'orifice (16) et d'autre part au moyen d'apport de fluide de gonflage.

4. Anneau chirurgical (1) selon l'une des revendications 1 à 3 **caractérisé en ce que** le moyen de mise en communication (14) forme un logement (18) qui entoure au moins une partie dudit moyen d'apport de fluide de gonflage.

5. Anneau chirurgical (1) selon l'une des revendications précédentes **caractérisé en ce que** la ceinture (2) comprend une face interne (2A) présentant une forme sensiblement complémentaire de celle de la face externe (10B) de la bande gonflable souple (10) contre laquelle ladite ceinture (2) est rapportée.

6. Anneau chirurgical (1) selon les revendications 2, 4 et 5 **caractérisé en ce que** la ceinture (2) est formée d'une pièce d'un seul tenant comprenant d'une part, sur sa face interne (2A), l'orifice (16) et d'autre part, dans le prolongement de l'une de ses extrémités, le conduit (18), de manière à former une ceinture (2) monobloc.

7. Anneau chirurgical (1) selon la revendication 2 **caractérisé en ce que** la bande gonflable souple (10) comprend au moins deux chambres de serrage annulaire (12, 13) qui s'étendent sensiblement parallèlement l'une à l'autre, l'orifice (16) de la ceinture (2) permettant la communication fluidique entre la bande gonflable souple (10) et le moyen d'apport de fluide de gonflage.

8. Anneau chirurgical (1) selon la revendication 7 **caractérisé en ce que** l'orifice (16) de la ceinture (2) assure la communication fluidique entre chacune des chambres de serrage (12, 13).

9. Anneau chirurgical (1) selon l'une des revendications précédentes **caractérisé en ce que** la bande gonflable souple (10) est formée d'une pièce d'un seul tenant de manière à former une bande gonflable souple (10) monobloc.

10. Procédé de fabrication d'un anneau chirurgical implantable (1) dans un corps humain ou animal, ledit anneau (1) étant destiné à venir entourer un organe biologique constituant une poche ou un conduit en vue de modifier la section de passage dudit organe, ledit procédé comprenant une étape de fabrication d'une ceinture (2) comportant au moins un moyen de verrouillage (3) dudit anneau (1) en position fonctionnelle fermée autour dudit organe biologique, une étape de fabrication d'une bande gonflable souple (10) comprenant au moins une chambre de serrage annulaire (12) destinée à recevoir un fluide de gonflage et comportant au moins une ouverture (11) permettant l'apport en son sein du fluide de gonflage, ainsi qu'une étape d'assemblage de la ceinture (2) avec la bande gonflable souple (10), ladite ceinture (2) étant rapportée contre la bande gonflable souple (10) et fixée à cette dernière, l'étape de fabrication de la ceinture (2) comprend une sous-étape de fabrication d'un moyen de mise en communication (14) de ladite au moins une ouverture (11) de la bande gonflable souple (10) avec un moyen d'apport de fluide de gonflage, ledit procédé de fabrication étant **caractérisé en ce que** le moyen de mise en communication (14) fabriqué comprend un moyen de fixation (19) conçu pour coopérer avec l'ouverture (11) de la bande gonflable souple (10) afin de contribuer, au moins localement à la solidarisation de la ceinture (2) à la bande gonflable souple (10) le moyen de fixation (19) fabriqué se présentant sous la forme d'un bouton (19A) dont la configuration est sensiblement complémentaire de celle de l'ouverture (11) de la bande gonflable souple (10), ladite ouverture (11) formant une boutonnière conçue pour accueillir ledit moyen de fixation (19).

11. Procédé de fabrication selon la revendication 10 **caractérisé en ce que** l'étape de fabrication de la ceinture (2) comprend une sous-étape de fabrication de la face interne (2A) de ladite ceinture (2), ladite face interne (2A) présentant une forme sensiblement complémentaire de celle de la face externe (10B) de la bande gonflable souple (10) contre laquelle ladite face interne (2A) de la ceinture (2) est rapportée, ladite face externe (10B) étant fabriquée au cours de l'étape de fabrication de ladite bande gonflable (10).

12. Procédé de fabrication selon la revendication 10 ou 11 **caractérisé en ce que** l'étape de fabrication de la ceinture (2) se fait par moulage d'une pièce d'un seul tenant de manière à former une ceinture (2) monobloc comprenant d'une part sur sa face interne (2A) un orifice (16) permettant la communication fluidique avec ladite ouverture (11) de la bande gonflable souple (10) et comprenant d'autre part dans son prolongement le moyen de mise en communication (14) formant un conduit (17), ledit conduit (17) étant connecté d'une part à l'orifice (16) et d'autre part au moyen d'apport de fluide de gonflage.

13. Procédé de fabrication selon l'une des revendications 10 à 12 **caractérisé en ce que** l'étape de fabrication de la bande gonflable souple (10) se fait par moulage d'une pièce d'un seul tenant de manière à former une bande gonflable souple (10) monobloc.

## Patentansprüche

1. Chirurgischer Ring (1), der in einen menschlichen oder tierischen Körper implantiert werden kann, wobei der Ring (1) ausgelegt ist, um ein biologisches Organ zu umgeben, das einen Beutel oder eine Leitung darstellt, um die Durchlassöffnung des Organs zu modifizieren, wobei der Ring (1) Folgendes umfasst
- einerseits einen Gurt (2), der mindestens ein Verriegelungsmittel (3) des Rings (1) umfasst, der in der Betriebsposition um das biologische Organ geschlossen ist, der ein Mittel zur Verbindung (14) der mindestens einen Öffnung (11) des flexiblen quellbaren Bands (10) mit einem Mittel (15) zur Zufuhr von Quellflüssigkeit umfasst, und
- andererseits ein flexibles quellbares Band (10), das mindestens eine ringförmige Klemmkammer (12) umfasst, die ausgelegt ist, um eine Quellflüssigkeit zu erhalten und mindestens die Öffnung (11) umfasst, die die Zufuhr in ihr Inneres der Quellflüssigkeit ermöglicht, wobei der Gurt (2) an das flexible quellbare Band (10) aufgesetzt und an dieses Letztere befestigt ist.
- wobei der Ring (1) **dadurch gekennzeichnet ist, dass** das Mittel zur Verbindung (14) ein Befestigungsmittel (19) umfasst, das entworfen ist, um mit der Öffnung (11) des flexiblen quellbaren Bands (10) zusammenzuarbeiten, um, mindestens lokal, zur festen Verbindung des Gurts (2) an das flexible quellbare Band (10) beizutragen, wobei das Befestigungsmittel (19) die Form eines Knopfes (19A) aufweist, dessen Konfiguration im Wesentlichen komplementär zu derjenigen der Öffnung (11) des flexiblen quellbaren Bands (10) ist, wobei die Öffnung (11) ein Knopfloch bildet, das entworfen ist, um das Befestigungsmittel (19) aufzunehmen.

2. Chirurgischer Ring (1) nach Anspruch 1, **dadurch gekennzeichnet, dass** das Mittel zur Verbindung (14) ein Loch (16) umfasst, das die fluidische Kommunikation mit der Öffnung (11) des flexiblen quellbaren Bands (10) sicherstellt.

3. Chirurgischer Ring (1) nach Anspruch 1 oder 2, **dadurch gekennzeichnet, dass** das Mittel zur Verbindung (14) eine Leitung (17) umfasst, die einerseits mit dem Loch (16) verbunden ist und andererseits mit dem Mittel zur Zufuhr von Quellflüssigkeit.

4. Chirurgischer Ring (1) nach einem der Ansprüche 1 bis 3, **dadurch gekennzeichnet, dass** das Mittel zur Verbindung (14) ein Lager (18) bildet, das mindestens einen Teil des Mittels zum Zufuhr von Quellflüssigkeit umgibt.

5. Chirurgischer Ring (1) nach einem der vorhergehenden Ansprüche, **dadurch gekennzeichnet, dass** der Gurt (2) eine innere Seite (2A) umfasst, die eine im Wesentlichen komplementäre Form zur derjenigen der äußeren Seite (10B) des flexiblen quellbaren Bands (10) darstellt, an die der Gurt (2) aufgesetzt ist.

6. Chirurgischer Ring (1) nach Anspruch 2, 4 und 5, **dadurch gekennzeichnet, dass** der Gurt (2) aus einem integralen Stück gebildet ist, das einerseits, auf seiner inneren Seite (2A), das Loch (16) umfasst, und andererseits, in der Verlängerung eines seiner Enden, die Leitung (18), um einen Gurt (2) aus einem einzigen Stück zu bilden.

7. Chirurgischer Ring (1) nach Anspruch 2, **dadurch gekennzeichnet, dass** das flexible quellbare Band (10) mindestens zwei ringförmige Klemmkammern (12, 13) umfasst, die sich im Wesentlichen parallel zueinander erstrecken, wobei das Loch (16) des Gurts (2) die fluidische Kommunikation zwischen dem flexiblen quellbaren Band (10) und dem Mittel zur Zufuhr von Quellflüssigkeit ermöglicht.

8. Chirurgischer Ring (1) nach Anspruch 7, **dadurch gekennzeichnet, dass** das Loch (16) des Gurts (2) die fluidische Kommunikation zwischen jeder der Klemmkammern (12, 13) sicherstellt.

9. Chirurgischer Ring (1) nach einem der vorhergehenden Ansprüche, **dadurch gekennzeichnet, dass** das flexible quellbare Band (10) aus einem integralen Stück gebildet ist, um ein flexibles quellbares Band (2) aus einem einzigen Stück zu bilden.

10. Verfahren zur Herstellung eines chirurgischen Rings (1), der in einen menschlichen oder tierischen Körper implantiert werden kann, wobei der Ring (1) ausgelegt ist, um ein biologisches Organ zu umgeben, das einen Beutel oder eine Leitung darstellt, um die Durchlassöffnung des Organs zu modifizieren, wobei das Verfahren einen Schritt des Herstellens eines Gurts (2) umfasst, der mindestens ein Mittel zur Verriegelung (3) des Rings (1) umfasst, der in der Betriebsposition um das biologische Organ geschlossen ist, und einen Schritt des Herstellens eines flexiblen quellbaren Bands (10), das mindestens eine ringförmige Klemmkammer (12) umfasst, die ausgelegt ist, um eine Quellflüssigkeit zur erhalten und mindestens eine Öffnung (11) umfasst, die die Zufuhr in ihr Inneres der Quellflüssigkeit ermöglicht, sowie einen Schritt des Zusammenbauens des Gurts (2) mit dem flexiblen quellbaren Band (10), wobei der Gurt (2) an das flexible quellbare Band (10) aufgesetzt und an dieses Letztere befestigt ist, wobei der Schritt des Herstellens des Bands (2) einen Unterschritt des Herstellens eines Mittels zur Verbindung (14) der mindesten einen Öffnung (11) des flexiblen quellbaren Bands (10) mit einem Mittel (15) zur Zufuhr von Quellflüssigkeit umfasst, wobei das Verfahren zur Herstellung **dadurch gekennzeichnet ist, dass** das hergestellte Verbindungsmittel (14) ein Befestigungsmittel (19) umfasst, das entworfen ist, um mit der Öffnung (11) des flexiblen quellbaren Bands (10) zusammenzuarbeiten, um, mindestens lokal, zur festen Verbindung des Gurts (2) mit dem flexiblen quellbaren Band (10) beizutragen, wobei das hergestellte Befestigungsmittel (19) die Form eines Knopfes (19A) aufweist, dessen Konfiguration im Wesentlichen komplementär zu derjenigen der Öffnung (11) des flexiblen quellbaren Bands (10) ist, wobei die Öffnung (11) ein Knopfloch bildet, das entworfen ist, um das Befestigungsmittel (19) aufzunehmen.

11. Verfahren zur Herstellung nach Anspruch 10, **dadurch gekennzeichnet, dass** der Schritt des Herstellens des Gurts (2) einen Unterschritt des Herstellens der inneren Seite (2A) des Gurts (2) umfasst, wobei die innere Seite (2A) eine im Wesentlichen komplementäre Form zur derjenigen der äußeren Seite (10B) des flexiblen quellbaren Bands (10) darstellt, an die die innere Seite (2A) des Gurts (2) aufgesetzt ist, wobei die äußere Seite (10B) im Laufe des Schritts des Herstellens des quellbaren Bands (10) hergestellt wird.

12. Verfahren zur Herstellung nach Anspruch 10 oder 11, **dadurch gekennzeichnet, dass** der Schritt des Herstellens des Bands (2) durch das Formen eines integralen Stücks erfolgt, um einen Gurt (2) aus einem einzigen Stück zu bilden, der einerseits, auf seiner inneren Seite (2A), ein Loch (16) umfasst, das die fluidische Kommunikation mit der Öffnung (11) des flexiblen quellbaren Bands (10) ermöglicht und andererseits in seiner Verlängerung das Mittel zur Verbindung (14) umfasst, das eine Leitung (17) bildet, wobei die Leitung (17) einerseits mit dem Loch (16) und andererseits mit dem Mittel zur Zufuhr von Quellflüssigkeit verbunden ist.

13. Verfahren nach einem der Ansprüche 10 bis 12, **dadurch gekennzeichnet, dass** der Schritt des Herstellens des flexiblen quellbaren Bands (10) durch das Formen eines integralen Stücks erfolgt, um ein flexibles quellbares Band (10) aus einem einzigen Stück zu bilden.

## Claims

1. Implantable surgical ring (1) in a human or animal body, said ring (1) being designed to surround a biological organ constituting a pocket or a conduit for modifying the passage section of said body, said ring (1) comprising-,:
- firstly a belt (2) having at least one locking means (3) of said ring (1) in functional closed position around said biological organ, comprising means of communication (14) of said at least one opening (11) of the inflatable flexible strip (10) with a means of supply of inflation fluid (15), and
- secondly a flexible inflatable belt (10) comprising at least one annular clamping chamber (12) for receiving an inflation fluid and comprising at least said opening (11) allowing the flow therein of the inflation fluid, said belt (2) being brought to the inflatable flexible strip (10) and fixed to it;
- said ring (1) being **characterised in that** said means of communication (14) comprises a fastening means (19) adapted to cooperate with the opening (11) of the inflatable flexible strip (10) to contribute, at least locally, to the fastening of the belt (2) in the inflatable flexible strip (10), the securing means (19) being in the form of a button (19A) having a configuration substantially complementary to that of the opening (11) of the inflatable flexible strip (10), said opening (11) forming a slot adapted to accommodate said fastening means (19).

2. Surgical ring (1) according to claim 1 **characterised in that** the means of communication (14) comprises an aperture (16) which provides fluid communication with said opening (11) of the inflatable flexible strip (10).

3. Surgical ring (1) according to claim 1 or 2 **characterised in that** the means of communication (14) comprises a conduit (17) connected on the one hand to the aperture (16) and on the other to the means of supply of the inflating fluid (15).

4. Surgical ring (1) according to one of claims 1 to 3 **characterised in that** the means of communication (14) forms a housing (18) surrounding at least a portion of said means for supplying the inflation fluid.

5. Surgical ring (1) according to one of the preceding claims **characterised in that** the belt (2) comprises an internal face (2A) having a substantially complementary shape to the external face (10B) of the inflatable flexible strip (10) to which said belt (2) is attached.

6. Surgical ring (1) according to claims 2, 4 and 5 **characterised in that** the belt (2) is integrally formed in one piece comprising, on the one hand, on its internal face (2A), the aperture (16) and, on the other hand, in the extension of one of its ends, the conduit (18), so as to form an integral belt (2).

7. Surgical ring (1) according to claim 2 **characterised in that** the inflatable flexible strip (10) comprises at least two annular clamping chambers (12, 13) which extend substantially parallel to each other, the aperture (16) of the belt (2) permitting fluid communication between the inflatable flexible strip (10) and the means for supplying the inflation fluid (15).

8. Surgical ring (1) according to claim 7 **characterised in that** the aperture (16) of the belt (2) provides fluid communication between each of the clamping chambers (12, 13).

9. Surgical ring (1) according to one of the preceding claims **characterised in that** the inflatable flexible strip (10) is integrally formed in one piece so as to form an inflatable flexible integral strip (10).

10. A method of manufacturing an implantable surgical ring (1) in a human or animal body, said ring (1) being designed to surround a biological organ constituting a pocket or a conduit for modifying the passage section of said body, said method comprising a step of manufacturing a belt (2) having at least one locking means (3) of said ring (1) in a functional closed position around said organic body, a stage of manufacturing a flexible inflatable belt (10) comprising at least an annular clamping chamber (12) for receiving an inflation fluid and having at least one opening (11) allowing the flow therein of the inflation fluid, and a stage of assembling the inflatable belt (2) with the flexible strip (10), said belt (2) being attached to the inflatable flexible strip (10) and secured thereto, wherein the stage of manufacturing the belt (2) comprises a sub-step of producing the means for establishing communication (14) of said at least one opening (11) of the inflatable flexible strip (10) with a means of supply of deflating fluid, said manufacturing method being **characterised in that** the fabricated means of communication (14) comprises a fastening means (19) adapted to cooperate with the opening (11) of the inflatable flexible strip (10) to contribute, at least locally, to the fastening of the belt (2) to the inflatable flexible strip (10), the securing means (19) being made in the form of a button (19A) having a configuration substantially complementary to that of the opening (11) of the inflatable flexible strip (10), said opening (11) forming a slot adapted to accommodate said fastening means (19).

11. Production method according to claim 10 **characterised in that** the step of manufacturing the belt (2) comprises a sub-step of making the internal face (2A) of said belt (2), said internal face (2A) having a substantially complementary shape to the external face (10B) of the flexible inflatable belt (10) to which said internal surface (2A) of the belt (2) is attached, said outer face (10B) being produced during the manufacturing step of said inflatable belt (10).

12. Production method according to claim 10 or 11 **characterised in that** the step of manufacturing the belt (2) is made by moulding a one piece to form an integral belt (2) comprising, on the one hand, on its internal face (2A) an aperture (16) permitting fluid communication with said opening (11) of the inflatable flexible strip (10) and on the other hand in its extension a means of communication (14) forming a conduit (17), said conduit (17) being connected on the one hand to the aperture (16) and on the other to the means of inflation fluid supply.

13. Production method according to one of claims 10 to 12 **characterised in that** the step of manufacturing the inflatable flexible strip (10) is taken by moulding a one-piece to form a flexible inflatable integral strip (10).
